# EUROPEAN PATENT SPECIFICATION

(11) **EP 1 349 824 B1**
(45) Date of publication and mention of the grant of the patent: **26.10.2005**
(21) Application number: 01946662.2
(22) Date of filing: 21.06.2001
(51) Int. Cl.: C07C 51/265, C07C 63/26

(54) **METHOD FOR INCREASING OXIDATION REACTOR PRODUCTION CAPACITY**
VERFAHREN ZUR ERHÖHUNG DER OXIDATIONSREAKTORPRODUKTIONSKAPAZITÄT
PROCEDE PERMETTANT D'AUGMENTER LA CAPACITE DE PRODUCTION D'UN REACTEUR D'OXYDATION

(30) Priority: 10.01.2001 US 757458; 10.01.2001 US 757455; 11.01.2001 WO PCT/US01/00825; 11.01.2001 WO PCT/US01/00826; 19.06.2001 US 884184; 19.06.2001 US 884381
(43) Date of publication of application: 08.10.2003
(73) Proprietor: E.I. DU PONT DE NEMOURS AND COMPANY, Wilmington, Delaware 19898 (US)
(72) Inventor: TURNER, John Arthur, Stokesley, Yorkshire TS9 5AS (GB); HOUSLEY, Samuel Duncan, Cleveland TS15 0JG (GB)
(74) Representative: Cockerton, Bruce Roger
(86) International application number: PCT/US2001/019960
(87) International publication number: WO 2002/055467

(56) References cited:
- EP-A- 0 686 633
- US-A- 3 595 908
- US-A- 4 892 970
- US-A- 5 869 736

## Description

The present invention relates to a method for increasing the rated capacity of a commercial oxidation reactor. More particularly, the present invention is a method for debottlenecking a commercial reactor system of the type used for catalytic liquid phase oxidation of paraxylene to produce terephthalic acid.

Practically all terephthalic acid is produced on a commercial scale by catalytic, liquid phase, air oxidation of paraxylene. Commercial processes use acetic acid as a solvent and a multivalent heavy metal or metals as catalyst. Cobalt and manganese are the most widely used heavy metal catalysts, and bromine is used as a renewable source of free radicals in the process.

Acetic acid, air (molecular oxygen), paraxylene and catalyst are fed continuously into a back-mixed oxidation reactor that is maintained at from 175°C to 225°C and 1000-3000 kPa (i.e., 10-30 atm). The feed acetic acid:paraxylene ratio is typically less than 5:1. Air is added to the reactor in amounts in excess of the stoichiometric requirements for full conversion of the paraxylene to terephthalic acid to minimize formation of undesirable by-products, such as color formers. The oxidation reaction is exothermic, and heat is removed from the reactor by allowing the acetic acid solvent to vaporize. The corresponding vapor is condensed and most of the condensate is refluxed to the reactor, with some condensate being withdrawn to control water concentration in the system (two moles of water are formed per mole of paraxylene reacted to terephthalic acid). The reactor residence time is typically 30 minutes to 2 hours, depending on the process. Depending on oxidation reactor operating conditions, e.g., temperature, catalyst concentration and residence time, significant degradation of the solvent and precursor can occur, which, in turn, can increase the cost of operating the process.

The effluent, i.e., reaction product, from the oxidation reactor is a slurry of crude terephthalic acid (TA) crystals in acetic acid. A significant and undesirable impurity in the crude TA is 4-carboxy-benzaldehyde (4-CBA), which is incompletely oxidized paraxylene, although p-tolualdehyde and p-toluic acid can also be present along with undesirable color formers. The slurry of crude terephthalic acid crystals is further processed (e.g., purified by post-oxidation and/or hydrogenation) and recovered (e.g., by filtration, washing and drying) according to established methods.

The present invention provides a reliable and affordable method to increase the production capacity of a conventional terephthalic acid process by up to 100% by increasing the capacity of the oxidation reactor system, i.e., "debottlenecking" the reactor system. Debottlenecking is achieved according to the invention by effectively staging the oxidation reaction utilizing a first reaction zone, i.e., first reactor, followed by a second reaction zone, i.e., the existing, conventional reactor.

### SUMMARY OF THE INVENTION

The present invention is a method for increasing the production capacity of an oxidation reactor for catalytic liquid phase oxidation of paraxylene. A first reaction zone, or first reactor, is positioned upstream of the conventional reactor, and the method is accomplished according to the sequential steps of feeding the reactants, including a suitable solvent, which is acetic acid, to the first reaction zone at elevated pressure wherein the solvent ratio (i.e., the acetic acid:paraxylene mass ratio) and the uptake of oxygen are controlled such that any terephthalic acid which forms remains in solution, and then feeding the resulting reaction medium to the second, i.e., conventional, oxidation reaction zone.
The method comprises:
(a) forming a feed stream comprising acetic acid and oxidation catalyst at a pressure in the range of from at least about 2,000 kPa up to 20,000 kPa;
(b) oxygenating the feed stream;
(c) continuously and simultaneously feeding (1) the oxygenated feed stream and (2) paraxylene to the first reaction zone to form a reaction medium in which the acetic acid:paraxylene mass ratio is in the range of from 10-20:1;
(d) limiting the uptake of oxygen within the reaction medium in said first reaction zone to a value which is less than 50% of the oxygen required for full conversion of the paraxylene to terephthalic acid;
(e) feeding the reaction medium to a second reaction zone, the existing conventional reactor, while simultaneously reducing the pressure of the reaction medium to a value in the range of from 1,000 kPa to below 2,000 kPa.

Terephthalic acid resulting from the second reaction zone, which is typically a slurry of terephthalic acid crystals, can be further processed and recovered according to any convenient method.

The preferred acetic acid:paraxylene mass ratio for economy and process operability is from 13-16:1. The uptake of oxygen in the first reaction zone is limited to a value below 50% of the oxygen required for full conversion of the paraxylene present to terephthalic acid to prevent significant quantities of TA being formed and solids being precipitated. The oxygen uptake will preferably lie in the range of from 30-40% of the oxygen required for full conversion of the paraxylene present.

Oxygen uptake in the first reaction zone is controlled by one or more of the following methods: (i) maintaining oxygen supply within a predetermined range, (ii) maintaining catalyst concentration within a predetermined range, (iii) limiting the residence time (defined as the reactor liquid volume divided by the reactor feed rate) within the first reaction zone to less than about 6 minutes, but preferably less than 4 minutes, and (iv) optionally removing heat from, i.e., cooling, the reaction medium as it exits the first reaction zone to a temperature which is below about 210°C.

According to a preferred embodiment of the invention, oxygen is dissolved directly into a feed stream comprising acetic acid and oxidation catalyst, and the oxygenated feed stream is then fed continuously and simultaneously with paraxylene into the first oxidation reaction zone, which is a plug flow reaction zone. Immediately upon entering the first reaction zone the paraxylene is thoroughly mixed with the oxygenated acetic acid to thereby initiate the reaction. By controlling the oxygen supply, catalyst concentration, residence time and optionally the temperature of the first reaction zone, it is possible to control, i.e., limit, the uptake of oxygen within the reaction zone to a value which is less than 50% of the oxygen required for full conversion of the paraxylene present to terephthalic acid. The reaction medium from the first reaction zone is then fed to the second, conventional, existing reactor.

### BRIEF DESCRIPTION OF THE DRAWING(S)

Fig. 1 is a simplified schematic diagram of a preferred embodiment of the invention.
Fig. 2 is a simplified schematic diagram of an alternative to the process diagram shown in Fig. 1 wherein a back-mixed reactor is illustrated.

### DETAILED DESCRIPTION OF THE INVENTION

The present invention resides in the discovery that it is possible, when carrying out liquid phase catalytic oxidation of paraxylene in the presence of an acetic acid solvent, to effectively stage the oxidation reaction on a commercial scale into a first high pressure and high solvent ratio reaction zone followed by a second, more conventional, reaction zone and thereby substantially improve process capacity, efficiency and product quality. The present invention is particularly applicable to increasing the capacity of conventional oxidation reactors, i.e., "debottlenecking" commercially operating production units, whereby a first reaction zone can be positioned ahead of the conventional reactor, and the resulting output of the staged system can be increased by up to 70% conveniently and without substantial capital investment that would otherwise be required for a new and/or larger and/or redesigned conventional reactor.

According to a preferred embodiment of the invention, the first reaction zone is a plug flow reactor. The term "plug flow reactor" is used herein to define a generally elongated, or tubular, reaction zone in which rapid and thorough radial mixing of the reactants accurs as they flow through the tube or conduit. The invention, however, is intended to embrace any reactor configuration which approximates to a plug flow reaction zone. According to an alternate embodiment of the invention, the first reactor can be a back-mixed reactor, meaning a highly mixed reactor, such as, for example, a stirred tank or a bubble column reactor. For all reactor types which might characterize the first reaction zone according to the invention, the supply of oxygen thereto is essentially pure gaseous oxygen. The first reaction zone is further characterized by a relatively high acetic acid:paraxylene mass ratio in the range of from 10-20:1 and a relatively high pressure, e.g., in the range of from at least 2,000 kPa up to 20,000 kPa. The operating pressure of the first reactor is chosen such that there is no vapor phase present in the first reaction zone, i.e., the first reaction zone is non-boiling. The first reaction zone is optionally cooled to limit the temperature of the reaction medium as it exits the first reaction zone to less than 210°C.

The process is carried out in the presence of an oxidation catalyst system, which can be homogeneous or heterogeneous. A homogeneous catalyst is normally used and is selected from one or more heavy metal compounds, such as, for example, cobalt, manganese and/or zirconium compounds. In addition, the catalyst will normally also include an oxidation promoter such as bromine. The catalyst metals and oxidation promoter largely remain in solution throughout the process and are recovered and recycled as a solution, after product recovery, with fresh catalyst make-up.

The feed stream to the first reaction zone contains typical oxidation catalyst components (e.g., Co, Mn, Br), but diluted by a factor of about 3 to 5 relative to the catalyst concentration in recycle mother liquor from product recovery. The catalyst concentration is subsequently raised to more conventional catalyst concentration levels when and as solvent is vaporized and removed overhead in the second reaction zone. The total catalyst metals concentration in the first reaction zone will typically lie in the range 150 to 1,000 ppm w/w, whereas the catalyst metals concentration in the second reaction zone will typically lie in the range of from 500 to 3,000 ppm w/w. When using a Co and Mn metal catalyst system and depending on the water concentration, a total catalyst metals concentration in the first reaction zone of greater than 200 ppm w/w has been observed to give satisfactory activity and selectivity. Preferably, however, the total metals concentration in the first reaction zone should be greater than 250 ppm w/w.

The oxidation reaction is highly exothermic. Depending on the first reactor solvent ratio and oxygen uptake and without a means of cooling the reaction, the heat of reaction could raise the temperature of the reaction medium to a value in excess of 210°C. A first reactor exit temperature below 210°C is desirable to minimize acetic acid burn. The first reaction zone may therefore optionally include a cooling coil or other internal or external means for removing heat satisfactorily from the reactor (and reaction medium) to control the exit temperature of the reaction medium below 210°C.

Control of temperature, catalyst concentration, reactor residence time, and maintaining the oxygen supply to the first reaction zone within a predetermined range makes it possible to conveniently limit the uptake of oxygen within the reaction medium to a value which is less than 50% of the oxygen required for full conversion of the paraxylene present to terephthalic acid.

It is critical to avoid precipitation of solid terephthalic acid (TA) in the first reaction zone onto any cooling surfaces. TA formation is limited by limiting oxygen uptake, and TA precipitation is prevented within the first reaction zone by maintaining a high acetic acid:paraxylene ratio within the reaction medium and by selecting an appropriate coolant (e.g., boiling water) and cooling means that avoids cold spots from forming at any location within the reaction medium.

On exiting the first reaction zone, the pressure of the reaction medium is reduced simultaneously as it is fed to the existing conventional oxidation reactor. This reactor is typically a stirred tank reactor, but it could also be a bubble column reactor, for example. Pressure reduction can be conveniently accomplished by passing the reaction medium through one or a plurality of pressure letdown valves positioned about the periphery of the reactor. Best results have been obtained when the reaction medium is dispersed rapidly upon entering the second reactor. Rapid dispersion can be achieved by using established methods for dispersing paraxylene-containing feeds in conventional reactors. In a stirred tank reactor, for example, this would include injecting the reaction medium into the reactor below the liquid line in close proximity to the discharge from an agitator impeller. Rapid dispersion of the reaction medium can be achieved in a bubble column reactor by injecting the reaction medium in close proximity to the air feeds.

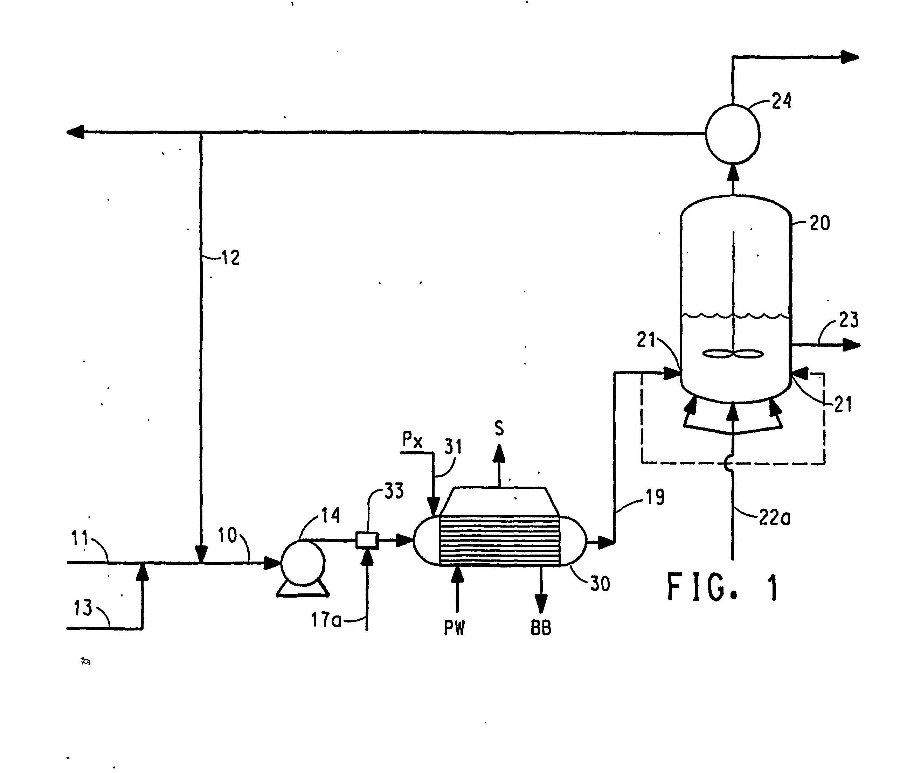

Referring now to the drawing, Fig. 1 is a simplified schematic diagram of a reactor system according to a preferred embodiment of the invention.

The invention is carried out by first forming a feed stream 10 comprising acetic acid, water and oxidation catalyst. In practice the feed stream will comprise a mixture of (i) recycled acetic acid, recycled mother liquor and catalyst, line 11, (ii) reactor condensate from the second reactor, line 12, and (iii) fresh acetic acid make-up, line 13. The mixed feed stream will contain typical catalyst components (e.g., Co, Mn, Br), but diluted compared to their respective concentrations in the conventional oxidation reactor. Optionally, although not shown, catalyst concentration can be controlled in the first reaction zone by feeding a portion of the catalyst-containing mother liquor, line 11, recycled from another part of the process, directly into second reactor 20.

The mixed feed stream 10 will generally have a temperature in the range of from 130°C to 160°C, based on the temperature of the various components which form the feed stream. A temperature in the range of from 130°C to 160°C has been found to be satisfactory for initiating the oxidation reaction.

The pressure of mixed feed 10 is raised to a value in the range of at least, but generally in excess of, 2,000 kPa by any suitable pumping means 14. The pressure is chosen to ensure that all of the gaseous oxygen, introduced via line 17a, will be readily dissolved in the feed stream ahead of first reactor 30 as shown. The mixed feed stream with dissolved oxygen is then fed simultaneously and continuously into plug flow reactor 30 with paraxylene being fed via line 31, and the reaction is initiated. The paraxylene may optionally be pre-mixed with acetic acid solvent and the mixture fed via line 31.

While it is generally preferable to feed all the paraxylene to the first reactor 30, the option of bypassing a portion of the feed paraxylene directly to the second reactor 20 is included within the scope of the invention. In cases where a portion of paraxylene feed 31 is fed directly to second reactor 20, the resulting solvent:paraxylene mass ratio in the reaction medium in the first reactor will adjust upwardly in response to that portion of the paraxylene feed which bypasses the first reactor, and the resulting mass ratio may, therefore, reach a value in the range of from 80:1 up to values in the range of 100:1 and even higher..

Molecular oxygen is dissolved in the mixed feed stream using any convenient in-line mixing device 33 to achieve a concentration of dissolved oxygen in the mixed feed stream of up to 3.0% w/w. Mixing device 33 could be an in-line nozzle arranged to discharge oxygen directly into the feed stream. In-line static mixers (not shown) can also be positioned upstream of first reactor 30 to facilitate mixing.

It is also possible according to the invention to stage the introduction of oxygen, i.e., to introduce the oxygen at a plurality of locations along the length of first reaction zone 30. By staging oxygen injection, the maximum local dissolved oxygen concentration is reduced, which, in turn, allows reactor operating pressure to be reduced. Reducing reactor operating pressure reduces the cost of the reactor, feed pump, oxygen compressor and associated equipment.

Residence time of the reaction medium within plug flow reaction zone 30 is relatively short, i.e., less than 6 minutes.

The reactor 30 shown in Fig. 1 is a shell and tube design. The reaction medium flows through the tubes, while a coolant, e.g., pressurized water (PW), is introduced into the shell side where it boils and is removed as steam (S). A small water purge (boiler blowdown, BB) is taken to control impurity/residue build-up in the water system.

The temperature of the reaction medium as it exits first reactor 30 is maintained at below about 210°C by controlling the pressure of the produced steam, and hence its temperature. Controlling the process parameters as described according to the invention makes it possible to limit the uptake of oxygen within the reaction medium in the first reaction zone to a value which is less than 50% of the oxygen required for full conversion of the paraxylene to TA. Thus, paraxylene is converted in first reactor 30 primarily to TA intermediates, such as p-tolualdehyde, p-toluic acid and 4-CBA. Under the described process conditions the first reactor will not produce any solid TA.

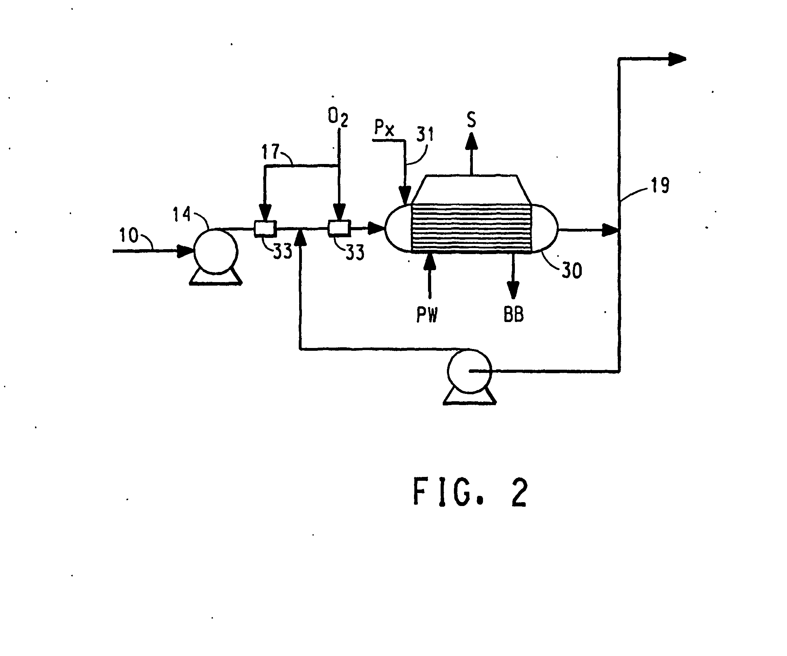

Although a shell and tube reactor design is shown in Fig. 1, reactor 30 can be any suitable reactor design with provisions for optional heat removal and optional multiple oxygen injection. For example, the reactor can have multiple tube passes, with optional oxygen injection into the reaction medium upstream of each tube pass. Alternatively, the reactor can be a single cooled or uncooled (adiabatic) stirred tank reactor with oxygen injection upstream of and/or into the reactor. Alternatively, the reactor can be a series of cooled or uncooloed stirred tank reactors with oxygen injection upstream of and/or into each reactor. As a further alternative, a back-mixed reactor can be employed, such as, for example, a pumped circulating loop reactor, with oxygen injection into the loop and optional heat removal from the loop as illustrated in Fig. 2.

The reaction medium exiting plug-flow first reactor 30 is fed via line 19 to a second reactor, i.e., oxidation zone, 20, which, as shown, is the conventional, continuously stirred tank reactor of the existing process which is the subject of debottlenecking. Simultaneously, the pressure of the reaction medium is reduced to a value in the range of from 1,000 kPa to below 2,000 kPa. Pressure reduction can be conveniently accomplished by passing the reaction medium through one or a plurality of pressure letdown valves or nozzles 21 positioned about the periphery of reactor 20 whereby the reaction medium is dispersed rapidly by injection into an agitator impeller region below the liquid line of the reactor. Process conditions within reactor 20, i.e., temperature, pressure, catalyst concentration and residence time, are within conventional ranges, although oxygen uptake is reduced for reduced oxidation intensity.

A fresh supply of air or oxygen-containing gas, line 22a, is introduced and rapidly dispersed into the reaction medium in second reactor 20 by any convenient means.

TA will precipitate to form a slurry within reactor 20, and it can be recovered from the reactor system via line 23 using conventional methods. Overhead vapor from reactor 20, which will necessarily contain some acetic acid and water, is condensed via condenser 24, and most of the condensate is returned, i.e., recycled, via line 12 for feed stream make-up to first reactor 30. A proportion of the acetic acid and water condensate stream (so-called water draw off) is diverted to a solvent dehydration system to remove the water of reaction. Optionally, but not shown, a portion of the condensate may be returned to reactor 20, to the reactor headspace, via a reflux slinger, and/or to the reaction zone, via a separate feed line or by mixing with the existing feed stream, line 19.

The invention provides an economical and reliable method for staging the TA oxidation reaction whereby the production capacity of a conventional single-stage oxidation reactor of the type found in many commercially operating terephthalic acid processes can be increased by up to 100%.

## Claims

1. A method for increasing the production capacity of a conventional oxidation reactor for catalytic liquid phase, air oxidation of paraxylene to terephthalic acid, said method comprising staging the reaction according to the following sequential steps:
(a) forming a feed stream comprising acetic acid and oxidation catalyst at an elevated pressure in the range of from 2,000 up to 20,000 kPa;
(b) oxygenating the feed stream;
(c) continuously and simultaneously feeding (1) the oxygenated feed stream and (2) paraxylene to a first reaction zone positioned upstream from said conventional oxidation reactor to form a reaction medium in which the acetic acid:paraxylene mass ratio is in the range of from 10-20:1 and reaction products are maintained in solution as they are formed;
(d) limiting the uptake of oxygen within the reaction medium in said first reaction zone to a value which is less than 50% of the oxygen required for full conversion of the paraxylene present to terephthalic acid;
(e) feeding the reaction medium to said conventional oxidation reactor while simultaneously reducing the pressure of the reaction medium to a value in the range of from 1,000 kPa to 2,000 kPa.

2. The process of Claim 1 in which said first reaction zone is a plug flow reactor or a back-mixed reactor.

3. The process of Claim 2 which comprises the additional steps of:
(a) vaporizing a portion of the acetic acid present in said conventional oxidation reactor;
(b) removing the vapor from the reactor overhead;
(c) condensing the vapor; and
(d) recycling some or all of the condensate to the feed stream.

4. The process of Claim 1 or Claim 3 which includes the additional step of diverting a portion of the paraxylene feed from the first reaction zone to said conventional reactor whereby the resulting solvent:paraxylene mass ratio in the reaction medium in the first reaction zone is adjusted upwardly in response to that portion of the paraxylene feed which bypasses the first reactor to achieve a corresponding value in excess of 25:1.

## Patentansprüche

1. Verfahren zum Erhöhen der Produktionskapazität eines konventionellen Oxidationsreaktors für die katalytische Luftoxidation in Flüssigphase von p-Xylol zu Terephthalsäure, wobei das Verfahren die schrittweise Ausführung der Reaktion entsprechend den folgenden hintereinander geschalteten Schritten umfasst:
(a) Erzeugen eines Essigsäure und Oxidationskatalysator aufweisenden Aufgabestroms bei einem erhöhten Druck im Bereich von 2.000 bis zu 20.000 kPa;
(b) den Aufgabestrom mit Sauerstoff versetzen;
(c) kontinuierliches und gleichzeitiges Zuführen des (1) mit Sauerstoff versetzten Aufgabestroms und des (2) p-Xylols zu einer ersten Reaktionszone, die zulaufseitig zu dem konventionellen Oxidationsreaktor angeordnet ist, um ein Reaktionsmedium zu erzeugen, worin das Masseverhältnis der Essigsäure:p-Xylol im Bereich von 10 bis 20:1 liegt und die Reaktionsprodukte bei ihrer Erzeugung in Lösung gehalten werden;
(d) Beschränken der Sauerstoffaufnahme in dem Reaktionsmedium in der ersten Reaktionszone auf einen Wert, der kleiner ist als 50% des zur vollständigen Umwandlung des vorhandenen p-Xylols zu Terephthalsäure erforderlichen Sauerstoffes ist;
(e) Zuführen des Reaktionsmediums zu dem konventionellen Oxidationsreaktor, während gleichzeitig der Druck des Reaktionsmediums bis auf einen Wert im Bereich von 1.000 kPa bis 2.000 kPa herabgesetzt wird.

2. Verfahren nach Anspruch 1, bei welchem die erste Reaktionszone ein Reaktionsapparat mit Pfropfenströmung oder ein rückvermischter Reaktor ist.

3. Verfahren nach Anspruch 2, welches Verfahren die zusätzlichen Schritte umfasst:
(a) Verdampfen eines Teils der in dem konventionellen Oxidationsreaktor vorhandenen Essigsäure;
(b) Entfernen des Dampfes aus dem Kopf des Reaktors;
(c) Kondensieren des Dampfes; und
(d) einen Teil oder das gesamte Kondensat in den Kreislauf zu dem Aufgabestrom zurückführen.

4. Verfahren nach Anspruch 1 oder 3, welches Verfahren den zusätzlichen Schritt des Ableitens eines Teils der p-Xylolbeschickung aus der ersten Reaktionszone zu dem konventionellen Reaktor einschließt, wodurch das resultierende Masseverhältnis von Lösemittel:p-Xylol in dem Reaktionsmedium in der ersten Reaktionszone in Reaktion auf denjenigen Teil der p-Xylolbeschickung nach oben geregelt wird, der um den ersten Reaktor umgeleitet wird, um einen entsprechenden Wert von über 25:1 zu erzielen.

## Revendications

1. Procédé pour l'augmentation de la capacité de production d'un réacteur d'oxydation conventionnel destiné à une oxydation par l'air, en phase liquide, catalytique de paraxylène en acide téréphtalique, ledit procédé comprenant l'élaboration en étapes de la réaction selon les étapes séquentielles suivantes:
(a) formation d'un courant d'alimentation comprenant de l'acide acétique et un catalyseur d'oxydation à une pression élevée dans l'intervalle de 2.000 jusqu'à 20.000 kPa;
(b) oxygénation du courant d'alimentation;
(c) introduction en continu et en simultané (1) du courant d'alimentation oxygéné et (2) de paraxylène dans une première zone de réaction positionnée en amont dudit réacteur d'oxydation conventionnel pour former un milieu de réaction dans lequel le rapport en masse d'acide acétique:paraxylène est dans l'intervalle de 10-20:1 et les produits de réaction sont maintenus en solution lorsqu'ils sont formés;
(d) limitation de l'absorption d'oxygène dans le milieu de réaction dans ladite première zone de réaction à une valeur qui est inférieure à 50% de l'oxygène requis pour une conversion complète du paraxylène présent en acide téréphtalique;
(e) introduction du milieu de réaction dans ledit réacteur d'oxydation conventionnel tout en réduisant simultanément la pression du milieu de réaction à une valeur dans l'intervalle de 1.000 kPa à 2.000 kPa.

2. Procédé suivant la revendication 1, dans lequel ladite première zone de réaction est un réacteur à écoulement piston ou un réacteur mélangé à contre-courant.

3. Procédé suivant la revendication 2, qui comprend les étapes supplémentaires:
(a) d'évaporation d'une portion de l'acide acétique présent dans ledit réacteur d'oxydation conventionnel;
(b) de retrait de la vapeur par le haut du réacteur;
(c) de condensation de la vapeur; et
(d) de recyclage d'une partie ou de la totalité du produit de condensation vers le courant d'alimentation.

4. Procédé suivant la revendication 1 ou la revendication 3, qui inclut l'étape supplémentaire de détournement d'une portion de l'alimentation de paraxylène depuis la première zone de réaction vers ledit réacteur conventionnel, en conséquence de quoi le rapport en masse de solvant:paraxylène résultant dans le milieu de réaction dans la première zone de réaction est ajusté vers le haut en réponse à cette portion de l'alimentation de paraxylène qui contourne le premier réacteur pour atteindre une valeur correspondante dépassant 25:1.
